# EUROPEAN PATENT APPLICATION

(11) **EP 2 253 285 A1**
(43) Date of publication of application: **24.11.2010**
(21) Application number: 08868517.7
(22) Date of filing: 24.12.2008
(51) Int. Cl.: A61B 17/58, F16B 7/04

(54) **INTRAMEDULLARY NAIL, AND CONTROL MEMBER USED IN THE NAIL**

(30) Priority: 28.12.2007 JP 2007338714
(71) Applicant: Groupe Lepine, 69730 Genay (FR)
(72) Inventor: TABE, Kosuke, Fukuoka 814-0111 (JP)
(74) Representative: Jeannet, Olivier
(86) International application number: PCT/JP2008/073450
(87) International publication number: WO 2009/084542

(57) **Abstract**

Disclosed is an intramedullary nail capable of controlling a plurality of engaging members (29, 33), if used, simultaneously with respect to a body. The intramedullary nail includes a rod body inserted into a medulla from one end of an extending bone and having a plurality of through holes (17, 19) intersecting the axis, and a plurality of engaging members (29, 33) individually inserted into the corresponding individual through holes (17, 19) of the body so that they can engage with the bone. Holes (21) are formed to extend from one end of the body to the individual through holes (17, 19). The intramedullary nail is the member which is inserted from the hole (21) and housed in the body inside, and is shaped such that its one end can contact with one of the engaging members (29, 33) and such that it can receive another engaging member. A control mechanism comprises a control member (1) capable of controlling the fixtures of the individual engaging members (29, 33) simultaneously with respect to the body.

## Description

### TECHNICAL FIELD

The present invention relates to an intramedullary nail and a control member used in the nail, and in particular, to an intramedullary nail and related items thereof comprising a rod-shaped main body inserted into an intramedullary part from one edge of an extended bone and having a plurality of through-holes intersecting with a shaft center thereof, a plurality of engaging members in which each of the corresponding members is inserted through each of the through-holes to be engageable with the bone.

### BACKGROUND ART

An intramedullary nail is used for a treatment such as a femoral fracture. Technologies of an intramedullary nail are as follows.

First, technologies described in Japanese Patent Laid-Open Publication Nos. H09-164151 and H10-66698 will be described. The intramedullary nail comprises a main body (referred to as an intramedullary stick in Japanese Patent Laid-Open Publication No. H09-164151, etc.) inserted from one edge into the intramedullary part of the extended bone in a femoral region; an engaging member (referred to as a neck screw in Japanese Patent Laid-Open Publication No. H09-164151, etc.) inserted through an inclined aperture formed in the main body and engaging with the femoral bone; and an adjusting member (referred to as a bore for example in Japanese Patent Laid-Open Publication No. H09-164151, etc.), which is capable of determining a degree of fixation of the engaging member to the main body. On the engaging member, grooves are formed along the extended direction. There are multiple types of adjusting members which further have different lengths. There are some adjusting members which are slidable in the grooves at one certain length and are engaged to prevent the members from rotating. At another length, there are adjusting members which are strongly contacted with pressure to the grooves and are fixed so that they can not slide.

Next, a technology described in Japanese Patent Laid-Open Publication No. 2000-342596 will be explained. Unlike Japanese Patent Laid-Open Publication Nos. H09-164151 and H10-66698, the main body (intramedullary rod in Japanese Patent Laid-Open Publication No. 2000-342596) is provided with a plurality of through-holes including a through-hole and a secondary through-hole, and a plurality of engaging members (lag screws in Japanese Patent Laid-Open Publication No. 2000-342596) are provided, each of which are inserted through the through-holes and secondary through-holes. Adjusting members with three types (different) of length which are called "end caps" are also provided. Two types of the length are the same as described in Japanese Patent Laid-Open Publication Nos. H09-164151, etc. When the shortest one is used, it is rotatable and freely slidable.

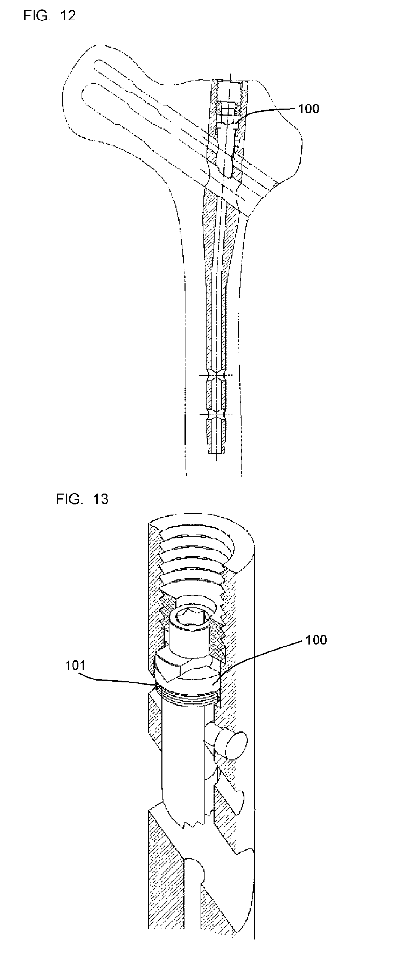

Further, as described in Japanese Patent Laid-Open Publication Nos. 2005-278819 and 2005-279140, there are some intramedullary nails, in which the mechanism 100 including the adjusting members have an elastic body 101 shown in Figs. 12 and 13 and it is devised to have an elasticity in the abutting direction that is the shaft center direction of the main body.

### PROBLEMS THAT THE INVENTION IS TO SOLVE

However, in the technologies described in either of the Japanese Patent Laid-Open Publication Nos. H09-164151, H10-66698, 2000-342596, 2005-278819 and 2005-279140, a degree of fixation for the main body of only one engaging member was controlled, even if engaging members were capable of being inserted into each of the plurality of through-holes of the main body. In other words, in the case where the engaging members were inserted in each of the plurality of through-holes of the main body, the degrees of fixation for the main body of the plurality of engaging members were not controlled at the same time. Therefore, one of the plurality of engaging members could rotate or slide, and then drop out.

Accordingly, the present invention aims to provide, in a case where a plurality of engaging members are used, an intramedullary nail which is capable of controlling the engaging members for the main body at the same time, and a control member used for the intramedullary nail.

### MEANS FOR SOLVING THE PROBLEMS

A first aspect in accordance with the present invention provides an intramedullary nail comprising a rod-shaped main body inserted from one edge of an extended bone into an intramedullary part and having a plurality of through-holes intersecting with a shaft center thereof, a plurality of engaging members each of which is inserted through each of the corresponding through-holes of the main body to be engageable with the bone, and a control mechanism including a control member in which degrees of fixation in relations between each of the plurality of engaging members and the main body are capable of being controlled at the same time, wherein a hole is formed linking from one end of the main body to each of the through-holes, and one end of the control member, which is inserted from the hole and then housed inside of the main body, has a shape which is capable of contacting with one of the plurality of engaging members and in which other engaging members are capable of being inserted through.

A second aspect in accordance with the present invention provides the intramedullary nail according to the first aspect, wherein the control mechanism includes an adjusting member which adjusts the degrees of fixation controlled by the control member by contacting and being compressed into the control member.

A third aspect in accordance with the present invention provides the intramedullary nail according to the second aspect, wherein the control member has a part in which distance between its surface and the shaft center becomes larger toward an aperture of the hole along the shaft center in a lateral surface of the control member upon its being housed, the main body has a part of an inner wall surface which follows a shape of a part of the control member in a state before the adjusting member is used, the adjusting member is used, thereby a contacting drag occurs between the part of the lateral surface of the control member and the part of the inner wall surface of the main body, and pressure occurring from the inner wall surface of the control member is applied to the other engaging members inserted through the through-hole so that the degree of fixation is adjusted.

A fourth aspect in accordance with the present invention provides the intramedullary nail according to the third aspect, wherein a shape of the other engaging member is in a shape prevented from rotating, with a surface contact or a multiple-site contact with the inner wall surface of the control member.

A fifth aspect in accordance with the present invention provides a control member used for an intramedullary nail comprising: a rod-shaped main body inserted from one edge of an extended bone into an intramedullary part and having a plurality of through-holes intersecting with a shaft center thereof, a plurality of engaging members each of which is inserted through each of the corresponding through-holes of the main body to be engageable with the bone, wherein a hole is formed linking from one end of the main body to each of the through-holes, one end of the control member, which is inserted from the hole and then housed into the inside of the main body, has a shape which is capable of contacting with one of the plurality of engaging members and in which other engaging member is capable of being inserting through, and degrees of fixation in relations between each of the plurality of engaging members and the main body are capable of being controlled at the same time.

### EFFECTS OF THE INVENTION

According to the present invention, degrees of fixation in a relationship between each the plurality of engaging members and the main body are capable of being controlled at the same time, which was not capable of being achieved traditionally, and thus prevents one of the plurality of engaging members from rotating, sliding and dropping out. Accordingly, an intramedullary nail with high reliability is obtained in both intraoperative and postoperative periods.

### BRIEF DESCRIPTION OF THE DRAWINGS

Fig, 1 is a perspective diagram showing a control member of an intramedullary nail according to an embodiment of the present invention.
Fig. 2 is an end view in an axial direction showing a state in which the control member of Fig. 1 is housed in a main body of the intramedullary nail.
Fig. 3 is an end view along a line III-III in Fig. 2.
Fig. 4 is a diagram showing a state in which a main lag screw is inserted from the state as shown in Fig. 2.
Fig. 5 is a diagram showing a state in which the main lag screw is inserted from the state as shown in Fig. 3.
Fig. 6 is a diagram showing a state in which a secondary lag screw is inserted from the state as shown in Fig. 4.
Fig. 7 is a diagram showing a state in which the secondary lag screw is inserted from the state as shown in Fig. 5.
Fig. 8 is a diagram showing a state in which a cap is screwed into an aperture 23 from the state shown in Fig. 6.
Fig. 9 is a diagram showing a state in which a cap is screwed into the aperture 23 from the state shown in Fig. 7.
Fig. 10 is a diagram showing a state in which the cap 35 enters the inside from the state shown in Fig. 8 by screwing into the inside, and in which a control member 1 controls degrees of fixation of the main lag screw 29 and the secondary lag screw 33 for the main body 15 at the same time.
Fig. 11 is a diagram showing a state in which the cap 35 enters the inside from the state shown in Fig. 9 by screwing into the inside, and in which the control member 1 controls a degree of fixation of the main lag screw 29 and the secondary lag screw 33 for the main body 15.
Fig. 12 is a diagram for explaining a traditional intramedullary nail.
Fig. 13 is a partial cross-sectional perspective view of an enlarged part of Fig. 12.

### REFERENCE NUMERALS

- 1: control member
- 11: flared portion
- 25: dent portion
- 15: main body of the intramedullary nail
- 21: hole
- 29: main lag screw
- 33: secondary lag screw

### BEST MODE FOR CARRYING OUT THE INVENTION

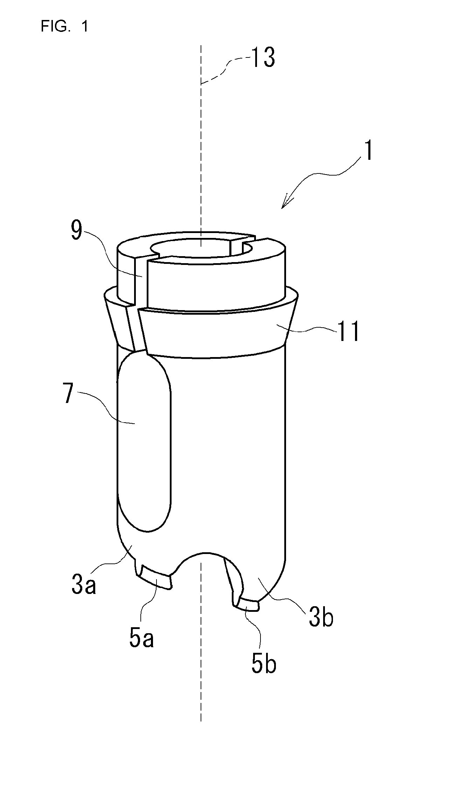

Fig. 1 is a perspective view showing a control member of an intramedullary nail according to an embodiment of the present invention.

The control member 1 called "a blocker," which controls the degree of fixation for a main body of an engaging member which is called a lag screw (not shown) in Fig. 1, is approximately in a column shape as a whole. At one end portion of the control member 1, two projecting portions 3a, 3b are formed so as to touch to the main lag screw. The projecting portions 3a, 3b are not at a position in a perpendicular direction for the direction in which the control member 1 extends, but at in an inclined position. This is because, as shown in Fig. 12, through-holes are formed to incline across the main body as shown in Fig. 12, the main lag screw is inserted through the main body, and as a result of the main lag screw being pushed in, each of the projecting portions 3a, 3b corresponds to the main lag screw. Here, a groove engaging portion 5a is formed in the projecting portion 3a, and a groove engaging portion 5b is formed in the projecting portion 3b. The groove engaging portions 5a, 5b are in a shape screwed in grooves formed in the main lag screw for preventing the lag screw from rotating.

Additionally, shapes of the groove engaging portions 5a, 5b and those of the projecting portion 3a, 3b are determined depending on the relationships with the shapes of the lag screws (including the grooves). Accordingly, it is not necessary to provide two projecting portions 3a and 3b, i.e., the number of the projecting portions may be 1, 3 or more. Further, the groove engaging portions 5a, 5b do not have to be formed in the whole projecting portions, but the groove engaging portions may be formed at the lag screws side and grooves at the projecting portion side. Then, as described in the prior art, various technologies may be used such as using an elastic body.

In the center region of the control member 1, an inserting-through pathway 7 is formed in the inclining direction but not in the perpendicular direction for the direction in which the control member 1 extends, and in which apertures at the sides are formed in a shape of elongate holes so that the secondary lag screw is inserted through. In addition, upper ends of the inserting-through pathway 7 in Fig. 1, corresponding to the location in the opposite direction of the projecting portions 3a, 3b are split in an upwards direction, and splits 9 with a predetermined width are provided.

In addition, a flared portion 11 is formed, which is located on the inserting-through pathway 7 and goes around except for the splitting portion 9 at the lateral surface of the control member 1. The flared portion 11 has a shape that extends and flares upward from the shaft center 13 in Fig. 1.

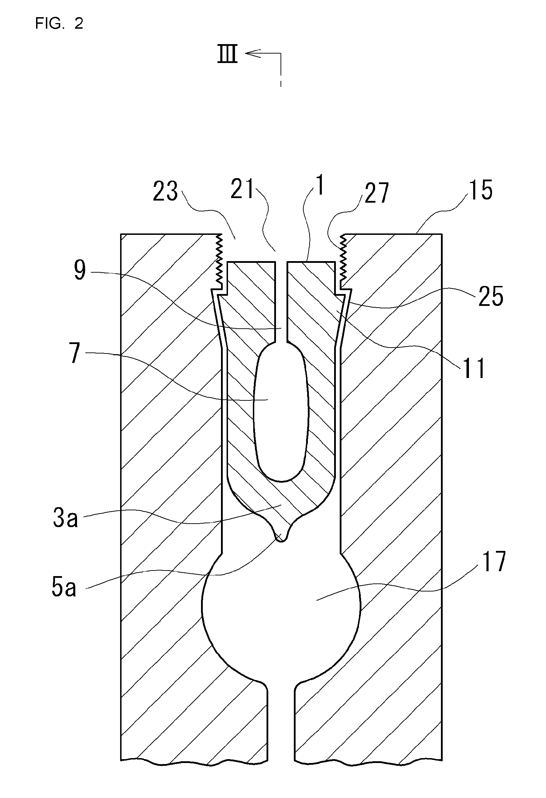

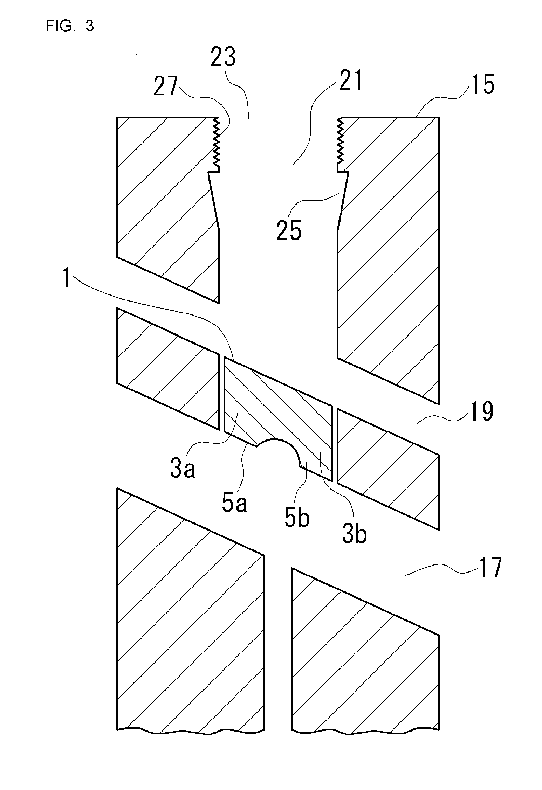

Fig. 2 is an end view in the axial direction showing where the control member of Fig. 1 is housed in the main body of the intramedullary nail. Fig. 3 is an end view along a line III-III in Fig. 2.

Referring to Fig. 2 and Fig. 3, a through-hole 17 and a through-hole 19 are formed in the main body of the intramedullary nail 15 as Fig. 12. A main lag screw is inserted through the through-hole 17, and a secondary lag screw is inserted through the through-hole 19. In the main body of the intramedullary nail 15, a hole 21 is formed, which passes from the upper end through the through-hole 19 and further through the through-hole 17. The control member 1 of Fig. 1 is inserted, putting the projecting portions 3a, 3b downward from the aperture 23 of the hole 21, and then is housed as shown in Figs. 2 and 3. The hole 21 has a shape following the lateral surface, in a state of the controlling member 1 as shown in Fig. 2. That is, in the hole 21, a dent portion 25 is formed to go around at the upper end denting with a shape following the shape of the flared portion 11. In the hole 21, a screw groove 27 which screws into the top edge is provided. In addition, as described later, positioning is performed so that the secondary lag screw is inserted through the inserting-through pathway 7 of the control member 1 while the secondary lag screw is inserted through the through-hole 19 of the main body of intramedullary nail 15.

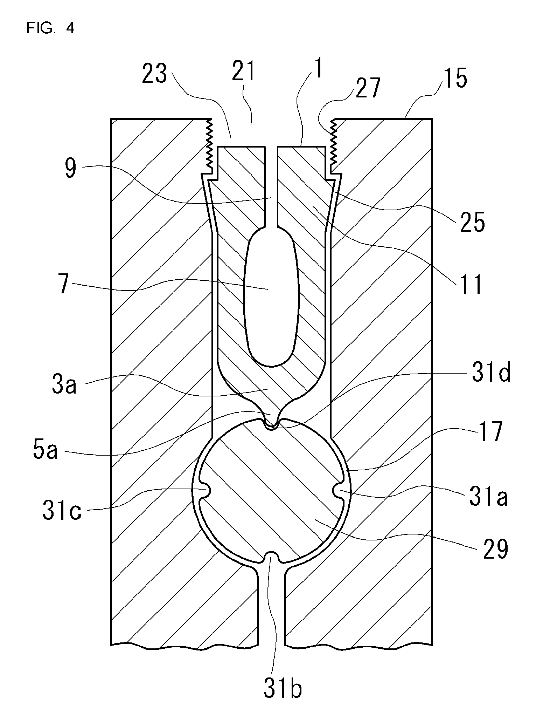

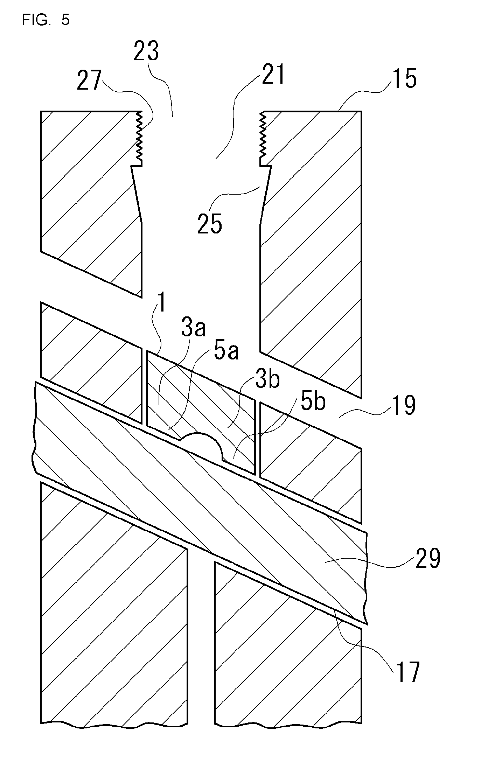

Fig. 4 is a diagram showing a state in which the main lag screw is inserted through from the state shown in Fig. 2. Fig. 5 is a diagram showing a state in which the main lag screw is inserted through from the state shown in Fig. 3.

As shown in Figs. 4 and 5, the main lag screw 29 is inserted. In the main lag screw 29, four grooves 31 a, 31 b, 31 c, 31 d are formed along the direction in which the circumference extends. In Fig. 4, the groove 31d and the groove engaging portions 5a, 5b of the control member 1 face each other. In addition, a positioning relationship between the groove 31d and the groove engaging portions 5a, 5b have been put in a state which can not prevent a rotation of the main lag screw in the state shown in Figs. 4 and 5. In this state, the main lag screw also may be prevented from rotating by means of positional relations of the members concerned.

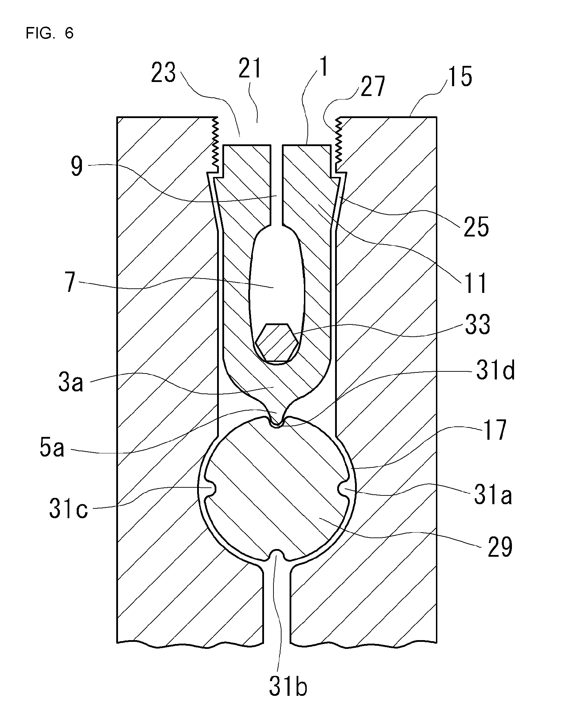

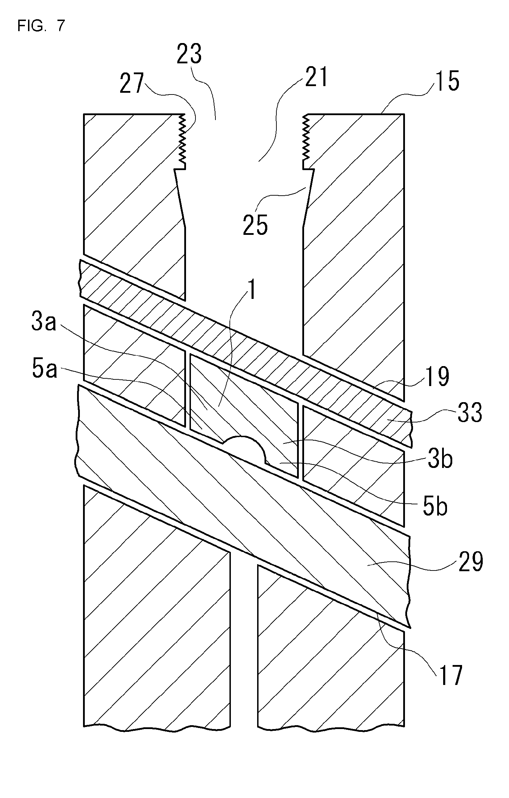

Fig. 6 is a diagram showing a state in which the secondary lag screw is inserted through from the state shown in Fig. 4. Fig. 7 is a diagram showing a state in which the secondary lag screw is inserted through from the state shown in Fig. 5.

As shown in Fig. 6 and Fig. 7, a secondary lag screw 33 is inserted. The secondary lag screw 33 here has a cross section with a hexagonal shape, and is arranged under the inserting-through pathway 7 of the control member 1.

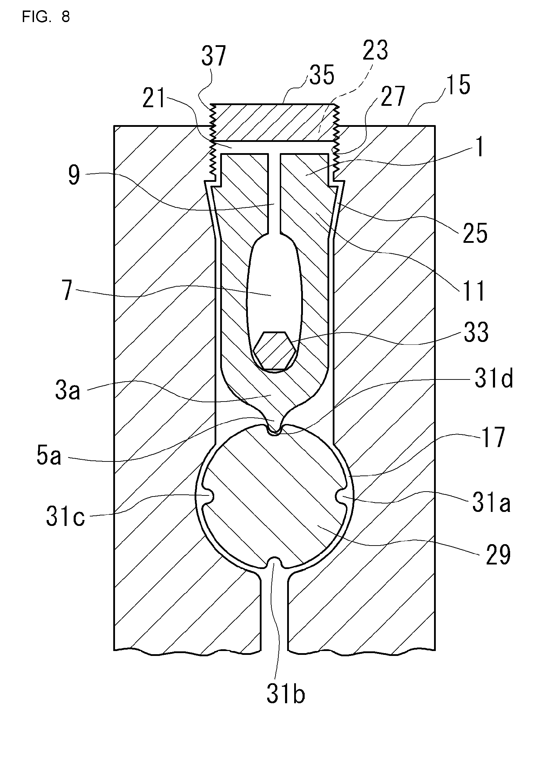

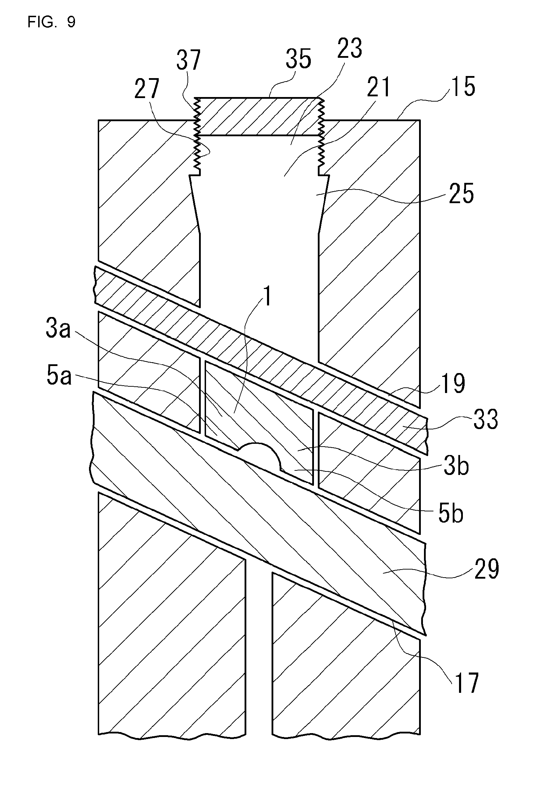

Fig. 8 is a diagram showing a state in which a cap is screwed into the aperture 23 from the state shown in Fig. 6. Fig. 9 is a diagram showing a state in which a cap is screwed into the aperture 23 from the state shown in Fig. 7.

As shown in Figs. 8 and 9, screw grooves 37, which screw with the screw grooves 27 formed at the top edge of the control member 1, are provided around a lateral side of the cap 35. These are screwed with each other, thereby to cover the hole 21.

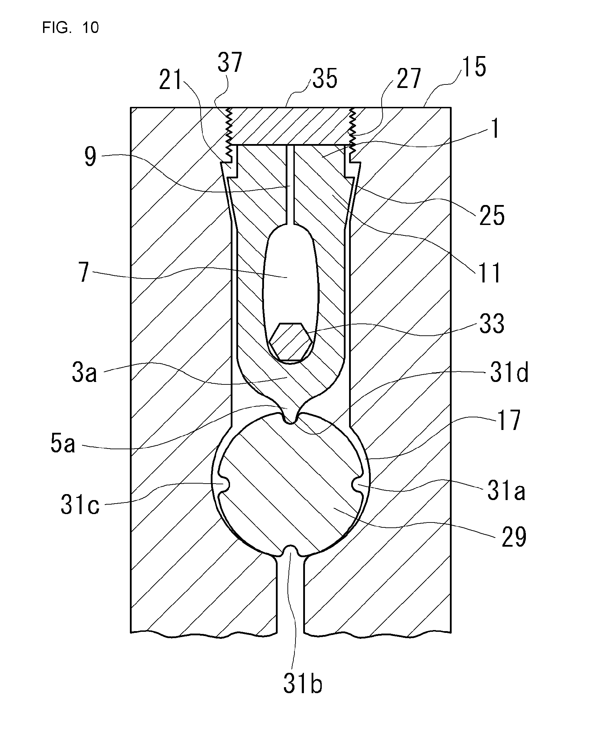

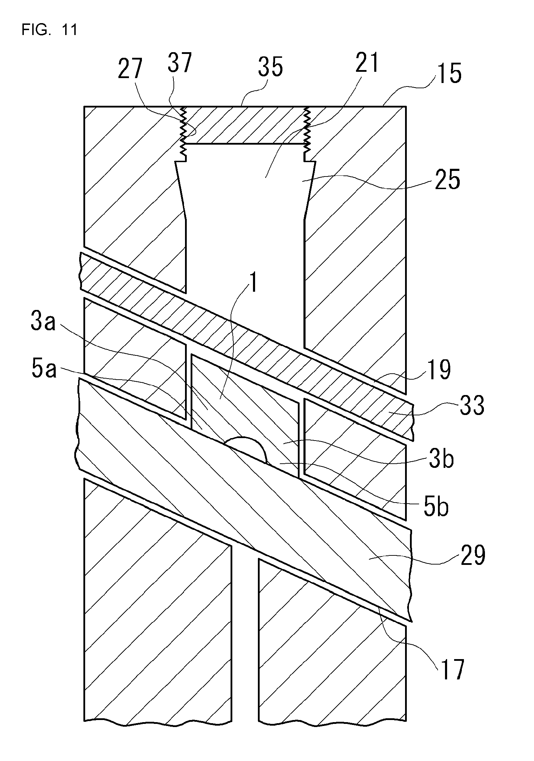

Fig. 10 is a diagram showing a state in which the cap 35 is driven into the inside by being screwed from the state shown in Fig. 8, and in which the control member 1 controls the degree of fixation of the main lag screw 29 and the secondary lag screw 33 for the main body 15 at the same time. Fig. 11 is a diagram showing a state in which the cap 35 enters the inside by being screwed from the state shown in Fig. 9, and in which the control member 1 controls the degree of fixation of the main lag screw 29 and the secondary lag screw 33 for the main body 15 at the same time.

In the states shown in Figs. 8 and 9, it is assumed that both the main lag screw 29 and the secondary lag screw 33 are rotatable and slidable. Hereinafter, it will be described that the control member 1 controls the degree of fixation of the main lag screw 29 for the main body 15 of the intramedullary nail, and then that the control member 1 controls the degree of fixation of the secondary lag screw 33 for the main body 15 of the intramedullary nail

In this state when the cap 35 enters into the inside by screwing, the main lag screw 29 becomes a state in which it is slidable but its rotation is prevented by engagement of the groove 31d and the groove engaging portions 5a, 5b. Further, great pressure is added between the grooves 31d and the groove engaging portions 5a, 5b (or, into the circumference of the projecting portions 3a, 3b and the main lag screw 29). Then, the control member 1 is presses to and brought into contact with the main lag screw 29 so as to be compressed into the lower side of the thorough-hole 17, and is therefore no longer slidable as shown in Figs. 10 and 11. Thus, the control member 1 controls the degree of fixation of the main lag screw 29 for the main body 15 of the intramedullary nail, by being compressed with the cap 35.

The secondary lag screw 33 is described as follows. If the cap 35 is compressed into the inside, the flared portion 11 of the control member 1 and the dent portion 25 of the hole 21 generate a contact drag and at the same time the control member 1 is caused to move downward, thereby maintaining the sliding state. Because the cross sectional area of the dent portion 25 becomes smaller towards the lower part and the cross sectional area of the flared portion 11 becomes larger towards the upper part as the control member 1 slides, an interval of the split 9 at the upper side of the control member 1 gradually narrows and the upper part of the inserting-through pathway 7 also gradually narrows. As a result, firstly, the cross-sectional shape of the secondary lag screw 33 is a hexagonal shape, thereby the surface or a plurality of angles contact with an inner wall surface of the inserting-through pathway 7, in a shape that the secondary lag screw 33 is prevented from rotating. This leads to a state that the secondary lag screw 33 is slidable but prevented from rotating. Further, if the interval of the splits 9 narrows down to be contacted, contact pressure between the circumference surface of the secondary lag screw 33 and the inner wall of the inserting-through pathway 7 becomes greater. Finally, as described in Figs 10 and 11, the secondary lag screw 33 is no longer slidable. Thus, the control member 1 controls the degree of fixation of the secondary lag screw 33 for the main body of the intramedullary nail 15 by being compressed with the cap 35.

In addition, the control member 1 controls the degree of fixation of the main lag screw 29 and the secondary lag screw 33 for the main body of the intramedullary nail 15 at the same time, as described above. However, the degrees of fixation of both lag screws for the main body of the intramedullary nail 15 do not have to be the same at the same timing. That is, for example, at the same timing, the rotation of the secondary lag screw may be in a state of being prevented while sliding of the main lag screw is prevented. The shape of the control member may be in a shape in which, assuming that such a situation occurs, then, even in a case that the lower end of the control member 1 abuts on the main lag screw to an extent even that the sliding strictly is prevented and thus, it is impossible to go downward, and the upper side thereof also dose not go downward despite sliding downward, the upper side of the control member narrows down toward the top edge and thus the interval of the splits 9 narrows down, thereby causing the secondary lag screw not to slide.

Further, the control member 1 does not have to be in a symmetrical shape and the inserting-through pathway 7 is not limited to be in the above shapes. In other words, there may be a case in which the wall surface of the control member 1 is pressed to and brought into contact with the wall surface of the secondary lag screw with pressure to the wall surface of the control member or to the through-hole 19 of the main body of the intramedullary nail 15.

In the above case, the flared portion 11 of the control member 1 is formed to go around the circumference, and the dent portion 25 is also formed to go around corresponding to the flared portion 11. However, each of these may be formed to go around a part of the circumferences.

Further in the above description, the secondary lag screw is set in a hexagonal shape. However, the secondary lag screw may be of other polygonal shapes. In addition, from the relationship with the shape of the controlling member 1, if inserting-through pathway is not an ellipse but a quadrangle, for example, the secondary lag screw may be, in addition to an ellipse, depending upon the degree of fixation required, a pair of planate parts which are not rotatable but still slidable, and a combination of curved parts such as a pair of circular arcs which are not rotatable nor slidable.

Further in the above description, after the control member 1 is inserted and housed into the hole 21 which is inside of the main body of the intramedullary nail 15, the control member 1 has a configuration in which is difficult for the control member 1 to drop out from the aperture 23, due to the shape relationship between the most flared upper end of flared portion 11 and the top edge of the dent portion 25. However, the configuration is not limited to this. That is, for example, the dent portion sides may not have a shape which is narrowed in shape toward the aperture, but in an opened shapes in which the cross-sectional areas are made the same or larger. The control member 1 may have a configuration in which the hole 21 is made to project on the inward side so that it is difficult for the control member 1 to drop out.

Further in the above description, the cap as an adjusting member is set to adjust the degree of fixation by the control member. However, there may be a case in which a center of the lower surface of the cap may be made to project so that the projected portion thereof and the secondary lag screw are made to be capable of contacting and engaging. Thereby, further control of the degree of fixation may be performed.

## Claims

1. An intramedullary nail, comprising:
a rod-shaped main body (15) inserted from one edge of an extended bone into an intramedullary part and having a plurality of through-holes (17, 19) intersecting with a shaft center thereof;
a plurality of engaging members (29, 33) each of which is inserted through each of the corresponding through-holes (17, 19) of the main body (15) to be engageable with the bone; and
a control mechanism including a control member (1) in which degrees of fixation in relationships between each of the plurality of engaging members and the main body (15) are capable of being controlled at the same time,
**characterized in that**
a hole (21) is formed linking from one end of the main body (15) to each of the through-holes (17, 19), and
one end of the control member (1), which is inserted from the hole (21) and then housed inside of the main body (15), has a shape which is capable of contacting with one of the plurality of engaging members (29) and in which other engaging members (33) are capable of being inserted through.

2. The intramedullary nail according to Claim 1, **characterized in that** the control mechanism includes an adjusting member which adjusts the degree of fixation controlled by the control member (1) by contacting and being compressed into the control member (1).

3. The intramedullary nail according to Claim 2,
**characterized in that**
the control member (1) has a part in which distance between its surface and the shaft center becomes larger toward an aperture of the hole along the shaft center in a lateral surface of the control member (1) upon its being housed;
the main body (15) has a part of an inner wall surface which follows a shape of a part of the control member (1) in a state before the adjusting member is used;
the adjusting member is used, thereby a contacting drag occurs between the part of the lateral surface of the control member (1) and the part of the inner wall surface of the main body (15); and
pressure occurring from the inner wall surface of the control member (1) is applied to the other engaging members inserted through the through-hole so that the degree of fixation is adjusted.

4. The intramedullary nail according to Claim 3, wherein a shape of the other engaging members is in a shape prevented from rotating, with a surface contact or a multiple-site contact with the inner wall surface of the control member (1).

5. A control member (1) used for an intramedullary nail, comprising:
a rod-shaped main body (15) inserted from one edge of an extended bone into an intramedullary part and having a plurality of through-holes intersecting with a shaft center thereof; and
a plurality of engaging members (29, 33) each of which is inserted through each of the corresponding through-holes (17, 19) of the main body (15) to be engageable with the bone,
**characterized in that**
a hole (21) is formed linking from one end of the main body (15) to each of the through-holes (17, 19),
one end of the control member (1), which is inserted from the hole (21) and then housed inside of the main body (15), has a shape which is capable of contacting with one of the plurality of engaging members and in which other engaging members are capable of being inserted through, and
degrees of fixation in relations between each of the plurality of engaging members and the main body (15) are capable of being controlled at the same time.
